# EUROPÄISCHE PATENTSCHRIFT

(11) **EP 3 669 620 B1**
(45) Veröffentlichungstag und Bekanntmachung des Hinweises auf die Patenterteilung: **10.11.2021**
(21) Anmeldenummer: 18746081.1
(22) Anmeldetag: 09.07.2018
(51) Int. Cl.: H05H 1/24

(54) **PLASMA-BEHANDLUNGSGERÄT FÜR EINE DIELEKTRISCHE BARRIERENENTLADUNG**
PLASMA TREATMENT UNIT FOR A DIELECTRIC BARRIER DISCHARGE
DISPOSITIF DE TRAITEMENT AU PLASMA POUR UNE DÉCHARGE À BARRIÈRE DIÉLECTRIQUE

(30) Priorität: 15.08.2017 DE 102017118568
(43) Veröffentlichungstag der Anmeldung: 24.06.2020
(73) Patentinhaber: Cinogy GmbH, 37115 Duderstadt (DE)
(72) Erfinder: WANDKE, Dirk, 37308 Heilbad Heiligenstadt (DE); HAHNL, Mirko, 37339 Berlingerode (DE); STORCK, Karl-Otto, 37115 Duderstadt (DE); TRUTWIG, Leonhard, 37115 Duderstadt (DE); RICKE, Melanie, 37191 Katlenburg-Lindau (DE)
(74) Vertreter: Gramm, Lins & Partner Patent- und Rechtsanwälte PartGmbB
(86) Internationale Anmeldenummer: PCT/DE2018/100625
(87) Internationale Veröffentlichungsnummer: WO 2019/034198

(56) Entgegenhaltungen:
- WO-A1-2010/103262
- DE-A1-102013 019 058
- US-A- 5 925 038
- US-A1- 2013 053 762
- US-A1- 2016 128 769
- US-A1- 2016 361 558
- US-A1- 2017 128 127

## Beschreibung

Die Erfindung betrifft ein Plasma-Behandlungsgerät mit einer in einem Gehäuse angeordneten Hochspannungsstufe zur Generierung von für die Erzeugung eines Plasmas geeigneten Hochspannungssignalen und einem mit der Hochspannungsstufe verbindbaren Kopfteil, in dem sich eine durch eine Dielektrikumsschicht abgeschirmte Elektrodenanordnung befindet.

Ein derartiges Plasma-Behandlungsgerät ist durch DE 10 2013 019 058 oder US 2016/0242269 A1 bekannt. Es handelt sich hierbei um ein Plasma-Behandlungsgerät, bei dem das Gehäuse ein Griffteil bildet, auf dem sich ein Kopfteil mit einer Kugelelektrode befindet. Die Kugelelektrode ist als starre Kugel auf einem elektrisch leitenden Material, nämlich Metall, ausgebildet und zumindest über den größten Teil der Kugeloberfläche mit einer dünnen Schicht aus einem Dielektrikum abgeschirmt. Der nicht abgeschirmte Teil der kugelförmigen Elektrode befindet sich innerhalb eines Gehäuseabschnitts des Kopfteils, aus dem die kugelförmige Elektrode lediglich mit einem kleinen Kugelabschnitt herausragt. Das Plasma-Behandlungsgerät dient dazu, mit der kugelförmigen Elektrode auf einer Oberfläche abrollbar zu sein, um so nach und nach eine größere Oberfläche mit dem von der Kugelelektrode erzeugten Plasmafeld behandeln zu können.

Es sind ferner aus der EP 1 628 688 B1 Elektrodenanordnungen bekannt, die stabförmig ausgebildet sind, um in beispielsweise eine Mundhöhle eingeführt werden zu können, um dort beispielsweise eine Zahnbehandlung vorzunehmen. Hierfür muss die Elektrodenanordnung wesentlich kleiner als die Mundhöhle sein.

Der Erfindung liegt die Aufgabe zugrunde, ein Plasma-Behandlungsgerät der eingangs erwähnten Art so auszubilden, dass neue Anwendungsgebiete für die Plasmabehandlung ermöglicht werden.

Zur Lösung dieser Aufgabe ist erfindungsgemäß ein Plasma-Behandlungsgerät der eingangs erwähnten Art dadurch gekennzeichnet, dass das Kopfteil ein an das Gehäuse anschließbares längliches Übergangsstück aufweist, an dessen nicht mit dem Gehäuse verbindbaren Ende ein Behandlungskopf angeordnet ist, dessen Abmessungen senkrecht zur Längsrichtung des Übergangsstücks die Abmessungen des Übergangsstücks deutlich übersteigen und dass in dem Behandlungskopf die Elektrodenanordnung eine räumlich geschlossene flexible Hülle um einen weichelastischen Kern bildet und an ihrer äußeren Mantelfläche durch eine dünne Schicht des flexiblen Dielektrikums abgedeckt ist, sodass der Behandlungskopf beim Einführen in ein Körperinneres die Form des umgebenen Gewebes im Körperinneren annehmen kann.

Durch das Plasma-Behandlungsgerät mit dem erfindungsgemäß ausgebildeten Behandlungskopf ist es möglich, eine Plasmabehandlung auch im Körperinneren mit einer hohen Effizienz durchzuführen. Durch die durch die Elektrodenanordnung gebildete räumlich geschlossene flexible Hülle und die Abdeckung dieser flexiblen Hülle durch ein flexibles Dielektrikum nach außen kann sich der Behandlungskopf an verschiedene Gegebenheiten in verschiedenen Körperhöhlen und - gängen anpassen. So ist beispielsweise eine Plasmabehandlung im Gehörgang möglich, um dort vorhandene Wundflächen, beispielsweise nach einer Ohrenoperation, schneller ausheilen zu lassen. Ferner können entzündliche Vorgänge im Gehörgang behandelt werden, in dem der Behandlungskopf, dessen Ausgangsabmessungen größer als die des Gehörgangs sind, in den Gehörgang eingeführt werden, wodurch der Behandlungskopf die Form des Gehörgangs annimmt.

In ähnlicher Weise kann eine Behandlung der Schleimhaut innerhalb der Nasengänge vorgenommen werden.

Mit dem erfindungsgemäßen Plasma-Behandlungsgerät können ferner Behandlungen in natürlich vorhandenen Körperhöhlen und -gängen jeglicher Art, beispielsweise im Enddarm, erfolgen. Möglich ist aber auch die Behandlung im Körperinneren durch künstliche Zugänge, wie sie bei minimal invasiven Operationen gelegt werden. Dementsprechend kann das Übergangsstück des Kopfteils insbesondere flexibel gestaltet werden und unterschiedliche Längen aufweisen. Insbesondere kann das Übergangsstück zum Behandlungskopf hin eine drahtähnliche oder schlauchähnliche Form aufweisen.

Bevorzugt ist ferner, dass das Gehäuse als Griffteil ausgebildet ist, mit dem das Kopfteil mit dem erfindungsgemäßen Behandlungskopf auswechselbar verbindbar ist. Das Kopfteil kann vorzugsweise auch als Einmal-Behandlungskopf steril verpackt sein, um nach der Behandlung ungefährlich entsorgt werden zu können. Der Aufwand für eine in vielen Fällen kritische Sterilisierung kann daher entfallen.

Bevorzugt ist eine Ausgangsform für den Behandlungskopf als Kugel oder als Kegelstumpf. Möglich sind aber auch andere Ausgangsformen, wie zylindrische Formen mit rundem oder eckigen Querschnitt, Würfelformen unterschiedlicher Größe usw.

Für den erfindungsgemäßen Behandlungskopf ist es vorteilhaft, wenn die Elektrodenanordnung durch eine gießfähige Kunststoffschicht gebildet ist, die durch leitende Zusätze leitfähig gemacht worden ist. Der Kunststoff der Elektrodenanordnung kann dabei von der gleichen Art sein wie das Dielektrikum, sodass sich die Elektrodenanordnung mit dem Dielektrikum leicht materialschlüssig verbinden.

Der weichelastische Kern besteht bevorzugt aus einem weichelastischen Kunststoffschaum oder einem weichelastischen Gel. Denkbar wäre auch die Ausbildung des weichelastischen Kerns durch ein eingeschlossenes Luft- oder Gasvolumen.

Da das die Mantelfläche der Elektrodenanordnung abdeckende Dielektrikum möglichst vollflächig an zu behandelndem Gewebe anliegt, ist es vorteilhaft, wenn die äußere Oberfläche des Dielektrikums eine äußere Struktur aufweist, die beim Anlegen an Wandungen im Körperinneren Freiräume zur Ausbildung des Plasmas bildet. Die äußere Struktur kann dabei durch als Abstandshalter wirkende Noppen, durch eine Gitterstruktur o. ä. gebildet sein.

Die Elektrodenanordnung kann aus einer einzigen geschlossenen Elektrodenschicht bestehen, die mit den Hochspannungssignalen versorgt wird und für die der zu behandelnde Körper als (floatende) Gegenelektrode fungiert. Die Elektrodenanordnung kann aber auch, beispielsweise durch ineinander verschachtelte Streifen, zwei Elektroden bilden, die vorzugsweise mit gegenphasigen Hochspannungssignalen gleicher Amplitude gespeist werden, sodass das umgebende Gewebe weiterhin als Gegen- oder Bezugselektrode fungiert, durch die beiden Elektroden jedoch immer eine resultierende Nullspannung mit Abstand von dem gebildeten Plasma entsteht.

Möglich ist ferner, die beiden Elektroden so zu speisen, dass eine das Hochspannungssignal erhält und die andere auf einem Bezugspotential liegt. In diesem Fall wird die Plasmabehandlung nicht so effizient durchgeführt wie bei der Verwendung des Körpers als Gegenelektrode. Im Einzelfall kann es jedoch sinnvoll sein, das Plasma-Behandlungsgerät selbst mit einem Bezugspotential zu betreiben.

Ein bevorzugtes Material für die Elektrodenanordnung und für das Dielektrikum ist jeweils ein Silikon, das dielektrische Eigenschaften hat, für die Elektrodenanordnung jedoch durch leitende Partikel leitfähig ausgebildet werden kann.

Die Erfindung soll im Folgenden anhand von in der Zeichnung dargestellten Ausführungsbeispielen näher erläutert werden. Es zeigen:
- Figur 1: Darstellungen eines Plasma-Behandlungsgeräts mit einem Behandlungskopf in Kugelform;
- Figur 2: Darstellungen wie in Figur 1 für das Plasma-Behandlungsgerät, bei dem der Behandlungskopf durch eine Einführung in eine Körperhöhle zu einem Kegelstumpf verformt ist.
- Figur 3: Darstellungen gemäß Figur 1 für ein Plasma-Behandlungsgerät, bei dem der Behandlungskopf in Kugelform auf seiner Außenseite Abstandshalter in Form von Noppen aufweist.
- Figur 4: Das Plasma-Behandlungsgerät gemäß Figur 3 vor dem Einführen in eine Körperhöhle (Figur 4a)) und nach der Einführung in eine Körperhöhle (Figur 4b)).

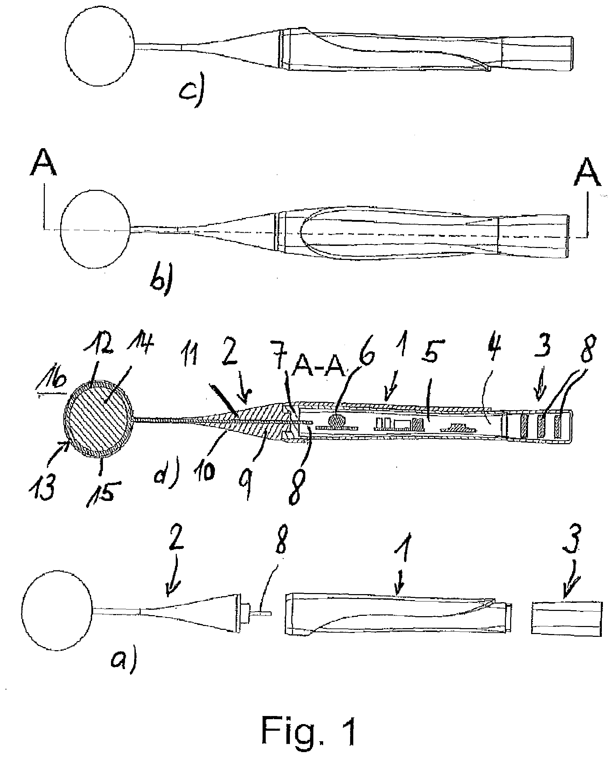

Figur 1 zeigt in vier Darstellungen ein erstes Ausführungsbeispiel eines Plasma-Behandlungsgeräts, wobei Figur 1a) eine Seitenansicht von drei noch nicht zusammengesteckten Geräteteilen, Figur 1b) eine Draufsicht auf das montierte Gerät, Figur 1c) eine Ansicht (von der bezüglich Figur 1a) gegenüber liegenden Seite) und Figur 1d) einen Schnitt durch das montierte Gerät gemäß der Schnittlinie A-A in Figur 1b) zeigt.

Das dargestellte Plasma-Behandlungsgerät besteht aus einem Griffteil 1, einem Kopfteil 2 und einem rückwärtigen Abschlussteil 3. Die Verbindungen zwischen Griffteil 1 und Kopfteil 2 sowie zwischen Griffteil 1 und rückwärtigem Abschlussteil 3 sind vorzugsweise Schnappverbindungen, können aber auch als Schraubverbindungen, Bajonettverbindungen o. ä. ausgebildet sein. Das Griffteil 1 weist eine leicht ergonomisch geformte Außenkontur auf, die das sichere Greifen des Plasma-Behandlungsgeräts ermöglichen soll. Das hohle Griffteil 1 weist einen Innenraum 4 auf, in dem sich eine elektronische Steuerung 5 befindet, die aus einer Gleichspannung Wechselspannungsimpulse generiert, die mit einer Spulenanordnung 6 in Hochspannungsimpulse übersetzt werden. Die Hochspannungsimpulse gelangen auf eine in ein stirnseitiges isolierendes Abschlussstück 7 eingesetzte, nicht näher dargestellte Buchse, über die die generierten Hochspannungsimpulse auf das Kopfteil 2 übertragen werden können.

Das rückwärtige Abschlussteil 3 umschließt drei Batterien 8, die durch Abnahme des rückwärtigen Abschlussteils 3 leicht zugänglich und auswechselbar sind. Das dargestellte Ausführungsbeispiel stellt somit ein Handgerät dar, das ohne Zuleitungskabel auskommt und daher bequem handhabbar ist. Im Rahmen der Erfindung ist es allerdings auch denkbar, das Gerät mit einer Stromzuführung über ein Kabel zu versorgen, wobei die Stromversorgung eine Gleichspannungsversorgung oder eine Wechselspannungsversorgung, beispielsweise aus dem öffentlichen Stromnetz, sein kann. Im letztgenannten Fall enthält die Steuerung 5 zweckmäßigerweise eine Gleichrichter- und Zerhackerstufe.

Das Kopfteil ragt mit einem Anschlussstift 8 aus dem Kopfteil heraus und in die Buchse des Abschlussstücks 7 des Griffteils 1. In dem Kopfteil 2 bildet ein Dielektrikum 9 ein Übergangsstück 10, das ein drahtförmiges Elektrodenstück 11 isolierend umgibt. Das drahtförmige Elektrodenstück 11 geht innerhalb eines sich an das Übergangsstück 10 anschließenden Behandlungskopfs in eine kugelförmige, geschlossene Hülle 12 über, sodass eine Elektrodenanordnung 13 durch den drahtförmigen Elektrodenabschnitt 11 und die hohle Hülle 12 gebildet wird. Die Hülle 12 umgibt einen weichelastischen Kern 14, beispielsweise aus einem weichelastischen Schaumstoff, einem weichelastischen Gel o. ä. Die Hülle 12 der Elektrodenanordnung 13 ist von einer geschlossenen Schicht 15 des Dielektrikums 9 vollständig umgeben, sodass sich durch die Elektrodenanordnung 13 immer nur eine dielektrisch behinderte Plasmaentladung ausbilden kann, in der kein resultierender Stromfluss von der Elektrodenanordnung 13 zu einer Gegenelektrode möglich ist. Die Schicht 15 kann an ihrer Oberfläche eine (nicht dargestellte) Struktur aufweisen, durch die zwischen der Oberfläche der Schicht 15 und dem umgebenden Gewebe Luft- bzw. Gasräume verbleiben, in denen das Plasma entstehen kann.

Aufgrund des weichelastischen Kerns 14 der dünnen flexiblen Hülle der Elektrodenanordnung 13 und der flexiblen dielektrischen Außenschicht 15 ist der so gebildete Behandlungskopf 16 leicht verformbar und kann sich der Form einer Körperhöhle oder eines Körperganges unproblematisch anpassen. Auf diese Weise gelingt eine intensive Plasmabehandlung in dem von dem Behandlungskopf 16 beaufschlagten Bereich des Gewebes.

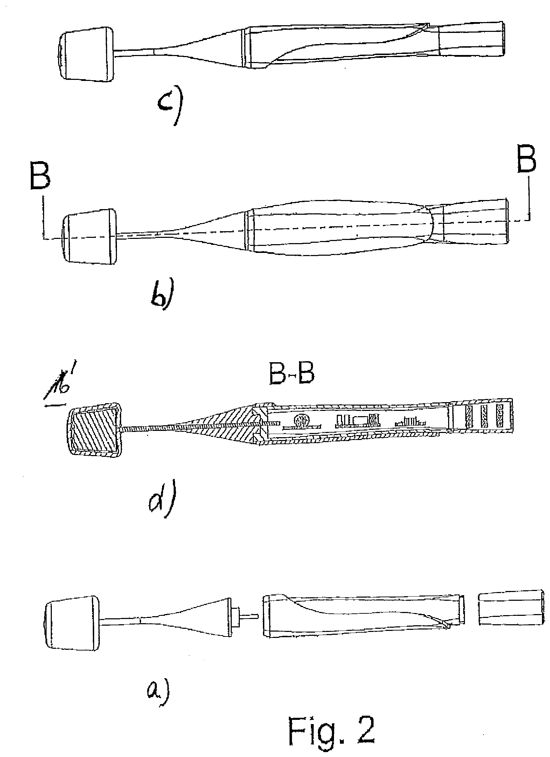

Figur 2 zeigt eine identische Ausführungsform, bei der ein in der gleichen Struktur aufgebauter Behandlungskopf 16' eine kegelstumpfförmige Ausgangsform aufweist. Diese Form des Behandlungskopfes 16' ist für die Einführung in Körpergänge, wie Gehörgang oder Nasengang, besonders geeignet.

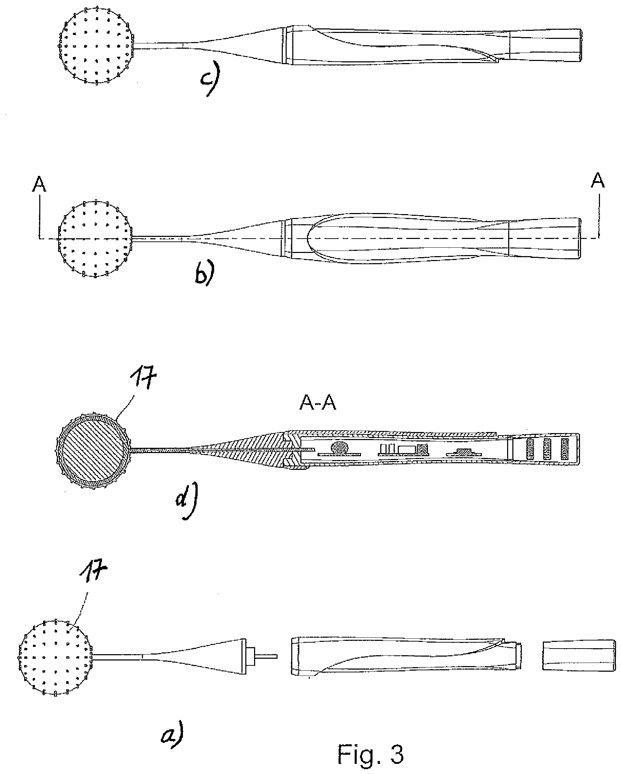

Das in Figur 3 dargestellte Plasma-Behandlungsgerät ist identisch zu dem Plasma-Behandlungsgerät gemäß Figur 1 ausgebildet, jedoch weist die kugelförmige Hülle 12 auf ihrer Außenseite Abstandshalter 17 auf, die hier in Form von Noppen ausgebildet sind. Die Abstandshalter 17 haben die Funktion, zwischen dem Körpergewebe und der Hülle 12 einen Luftraum zu gewährleisten, in dem das dielektrisch behinderte Plasma durch die Elektrodenanordnung 13 generiert werden kann.

Die Abstandshalter 17 sind gleichmäßig über die gesamte Kugeloberfläche der Hülle 12 verteilt angeordnet. Dadurch ist das Kopfteil 2 universell verwendbar. Für spezielle Anwendungsformen kann es auch sinnvoll sein, ein Kopfteil 2 zu verwenden, bei dem die Abstandshalter 17 nur abschnittsweise auf der Oberfläche der Hülle 12 angeordnet sind.

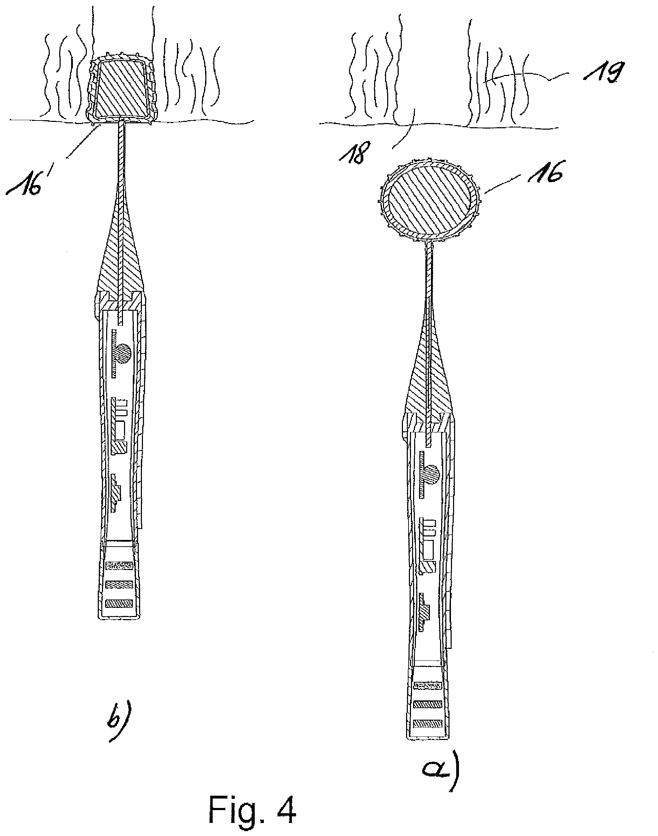

Figur 4 zeigt das Plasma-Behandlungsgerät gemäß Figur 3 vor dem Einführen in eine Körperöffnung 18, die von einem Körpergewebe 19 umgeben ist. Die Körperöffnung 18 kann eine natürliche Körperöffnung, wie ein Gehörgang, eine Nasenöffnung, eine Rektalöffnung o. ä., sein aber auch eine künstliche Öffnung oder ein künstlicher Zugang in Form eines Katheters oder eines Operationsschnitts sein. Figur 4b) verdeutlicht dabei, dass durch das Eindrücken des Kopfteils 2 die im Ruhezustand hohlkugelförmige Hülle 12 verformt wird, sodass sie beispielsweise, wie in Figur 4b) dargestellt, eine Kegelstumpfform annimmt, um so einen großflächigen Kontakt mit dem umgebenden Gewebe 19 herzustellen, das auf diese Weise mit dem sich in den Zwischenräumen zwischen den Abstandshaltern 17 ausbildenden dielektrisch behinderten Plasma intensiv behandelt werden kann. Auf diese Weise lassen sich beispielsweise operativ gelegte Zugänge, Operationsschnitte o. ä. behandeln, um Infektionen in diesem Bereich entgegenzuwirken, die Mikrozirkulation des Gewebes 19 im Bereich der Körperöffnung 18 anzuregen und den Heilungsprozess für die Operationswunde zu beschleunigen.

## Patentansprüche

1. Plasma-Behandlungsgerät mit einer in einem Gehäuse angeordneten Hochspannungsstufe (5,6) zur Generierung von für die Erzeugung eines Plasmas geeigneten Hochspannungssignalen und einem mit der Hochspannungsstufe (5,6) verbindbaren Kopfteil (2), in dem sich eine durch ein flexibles Dielektrikum (9) abgeschirmte Elektrodenanordnung (13) befindet, wobei das Kopfteil (2) ein an das Gehäuse anschließbares längliches Übergangsstück (10) aufweist, an dessen nicht mit dem Gehäuse verbindbaren Ende ein Behandlungskopf (16,16') angeordnet ist, dessen Abmessungen senkrecht zur Längsrichtung des Übergangsstücks (10) die Abmessungen des Übergangsstücks (10) deutlich übersteigen und
**dadurch gekennzeichnet,**
**dass** in dem Behandlungskopf (16,16') die Elektrodenanordnung (13) eine räumlich geschlossene flexible Hülle (12) um einen weichelastischen Kern (14) bildet und an ihrer äußeren Mantelfläche durch eine dünne Schicht (15) des flexiblen Dielektrikums (9) abgedeckt ist, sodass der Behandlungskopf (16, 16') geeignet ist, beim Einführen in ein Körperinneres, die Form des umgebenden Gewebes im Körperinneren anzunehmen.

2. Plasma-Behandlungsgerät nach Anspruch 1, **dadurch gekennzeichnet, dass** die dünne Schicht (15) des flexiblen Dielektrikums (9) eine äußere Struktur aufweist, die beim Anlegen an Wandungen im Körperinneren Freiräume zur Ausbildung des Plasmas bildet.

3. Plasma-Behandlungsgerät nach Anspruch 1 oder 2, **dadurch gekennzeichnet, dass** das Übergangsstück (10) flexibel ausgebildet ist.

4. Plasma-Behandlungsgerät nach einem der Ansprüche 1 bis 3, **dadurch gekennzeichnet, dass** das Übergangsstück (10) zum Behandlungskopf (16, 16') eine drahtähnliche Form aufweist.

5. Plasma-Behandlungsgerät nach einem der Ansprüche 1 bis 3, **dadurch gekennzeichnet, dass** das Übergangsstück (10) zum Behandlungskopf (16, 16') hin eine schlauchähnliche Form aufweist.

6. Plasma-Behandlungsgerät nach einem der Ansprüche 1 bis 5, **dadurch gekennzeichnet, dass** Gehäuse als Griffteil (1) ausgebildet ist, mit dem das Kopfteil (2) auswechselbar verbindbar ist.

7. Plasma-Behandlungsgerät nach einem der Ansprüche 1 bis 6, **dadurch gekennzeichnet, dass** der Behandlungskopf (16) eine Kugelform aufweist.

8. Plasma-Behandlungsgerät nach einem der Ansprüche 1 bis 6, **dadurch gekennzeichnet, dass** der Behandlungskopf (16') eine Kegelstumpfform aufweist.

## Claims

1. A plasma treatment unit having a high-voltage stage (5, 6) arranged in a housing for generating high-voltage signals suitable for the generation of a plasma and a head part (2) connectable to the high-voltage stage (5, 6), in which an electrode arrangement (13) shielded by a flexible dielectric material (9) is located, wherein the head part (2) comprises an oblong transition part (10) attachable to the housing, at the end thereof which is not connectable to the housing a treatment head (16, 16') is arranged, the dimensions of which perpendicular to the longitudinal direction of the transition part (10) significantly exceed the dimensions of the transition part (10), and **characterized in that** in the treatment head (16, 16'), the electrode arrangement (13) forms a spatially closed flexible envelope (12) around a soft-elastic core (14) and is covered on its outer lateral surface by a thin layer (15) of the flexible dielectric material (9), so that the treatment head (16, 16') is suitable, upon insertion into a body interior to assume the shape of the surrounding tissue in the body interior.

2. The plasma treatment unit as claimed in claim 1, **characterized in that** the thin layer (15) of the flexible dielectric material (9) comprises an outer structure, which forms free spaces for the formation of the plasma upon application to walls in the body interior.

3. The plasma treatment unit as claimed in claim 1 or 2, **characterized in that** the transition part (10) is formed to be flexible.

4. The plasma treatment unit as claimed in any one of claims 1 to 3, **characterized in that** the transition part (10) to the treatment head (16, 16') has a shape similar to wire.

5. The plasma treatment unit as claimed in any one of claims 1 to 3, **characterized in that** the transition part (10) to the treatment head (16, 16') has a shape similar to tubing.

6. The plasma treatment unit as claimed in any one of claims 1 to 5, **characterized in that** the housing is formed as a handle part (1), to which the head part (2) is replaceably connectable.

7. The plasma treatment unit as claimed in any one of claims 1 to 6, **characterized in that** the treatment head (16) has a ball shape.

8. The plasma treatment unit as claimed in any one of claims 1 to 6, **characterized in that** the treatment head (16') has a truncated cone shape.

## Revendications

1. Appareil de traitement au plasma, comprenant un étage haute tension (5, 6) disposé dans un boîtier et destiné à générer des signaux haute tension convenant à la génération d'un plasma, et une partie de tête (2) qui peut être reliée à l'étage haute tension (5, 6) et dans laquelle se trouve un ensemble d'électrode (13) protégé par un diélectrique flexible (9), la partie de tête (2) présentant une pièce de transition allongée (10) qui peut être raccordée au boîtier et à l'extrémité de laquelle, qui ne peut pas être reliée au boîtier, est disposée une tête de traitement (16, 16') dont les dimensions perpendiculaires à la direction longitudinale de la pièce de transition (10) dépassent nettement les dimensions de la pièce de transition (10), **caractérisé en ce que**, dans la tête de traitement (16, 16'), l'ensemble d'électrode (13) forme une gaine flexible (12) fermée dans l'espace autour d'un noyau élastique mou (14) et est recouvert, sur sa surface enveloppe extérieure, d'une couche mince (15) du diélectrique flexible (9), de sorte que la tête de traitement (16, 16') est apte à prendre la forme du tissu environnant à l'intérieur du corps lorsqu'elle est introduite dans l'intérieur du corps.

2. Appareil de traitement au plasma selon la revendication 1, **caractérisé en ce que** la couche mince (15) du diélectrique flexible (9) présente une structure extérieure qui, lorsqu'elle est appliquée contre des parois à l'intérieur du corps, forme des espaces libres pour la formation du plasma.

3. Appareil de traitement au plasma selon la revendication 1 ou 2, **caractérisé en ce que** la pièce de transition (10) est réalisée de façon flexible.

4. Appareil de traitement au plasma selon l'une des revendications 1 à 3, **caractérisé en ce que** la pièce de transition (10) vers la tête de traitement (16, 16') présente une forme similaire à un fil.

5. Appareil de traitement au plasma selon l'une des revendications 1 à 3, **caractérisé en ce que** la pièce de transition (10) vers la tête de traitement (16, 16') présente une forme similaire à un tuyau.

6. Appareil de traitement au plasma selon l'une des revendications 1 à 5, **caractérisé en ce que** le boîtier est réalisé sous la forme d'une partie formant poignée (1) à laquelle la partie de tête (2) peut être reliée de manière interchangeable.

7. Appareil de traitement au plasma selon l'une des revendications 1 à 6, **caractérisé en ce que** la tête de traitement (16) présente une forme sphérique.

8. Appareil de traitement au plasma selon l'une des revendications 1 à 6, **caractérisé en ce que** la tête de traitement (16') présente une forme tronconique.
